(19) Europäisches Patentamt
European Patent Office
Office européen des brevets

(11) Veröffentlichungsnummer: **0 304 534 A1**

(12) # EUROPÄISCHE PATENTANMELDUNG

(21) Anmeldenummer: **88101620.8**

(22) Anmeldetag: **04.02.88**

(51) Int. Cl.⁴: **C07D 403/12 , C07D 403/14 , C07D 237/04 , A61K 31/50**

Patentansprüche für folgende Vertragsstaaten: ES + GR.

(30) Priorität: **26.08.87 DE 3728491**

(43) Veröffentlichungstag der Anmeldung: **01.03.89 Patentblatt 89/09**

(84) Benannte Vertragsstaaten: **AT BE CH DE ES FR GB GR IT LI LU NL SE**

(71) Anmelder: **HEUMANN PHARMA GMBH & CO**
**18-28 Heideloffstrasse**
**D-8500 Nürnberg(DE)**

(72) Erfinder: **Schickaneder, Helmut, Dr. Dipl.-Chem.**
**Moosäcker 23**
**D-8501 Eckental(DE)**
Erfinder: **Mörsdorf, Peter, Dr. Dipl.-Chem.**
**Ziegelstrasse 64**
**D-8506 Langenzenn(DE)**
Erfinder: **Pfahlert, Volker, Dr.**
**Hummelsteinerweg 75**
**D-8500 Nürnberg(DE)**
Erfinder: **Engler, Heidrun, Dr.**
**Ringstrasse 23**
**D-8501 Cadolzburg(DE)**
Erfinder: **Ahrens, Kurt Henning, Dr.**
**Praterstrasse 9**
**D-8500 Nürnberg(DE)**

(74) Vertreter: **Kraus, Walter, Dr. et al**
**Patentanwälte Kraus, Weisert & Partner**
**Thomas-Wimmer-Ring 15**
**D-8000 München 22(DE)**

(54) **Dihydropyridazinon-Derivate, Verfahren zu ihrer Herstellung und diese Verbindungen enthaltende Arzneimittel.**

(57) Beschrieben werden neue Dihydropyridazinon-Derivate der allgemeinen Formel

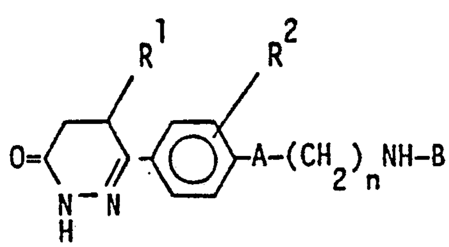

(I)

sowie ein Verfahren zu ihrer Herstellung und diese Verbindungen enthaltende Arzneimittel. Diese Verbindungen stellen wirksame positiv inotrope Substanzen mit einem verbesserten therapeutischen Profil dar.

**EP 0 304 534 A1**

# Dihydropyridazinon-Derivate, Verfahren zu ihrer Herstellung und diese Verbindungen enthaltende Arzneimittel

In der Behandlung der kongestiven Kardiomyopathie und der Herzinsuffizienz werden traditionell Herzglycoside, wie Digoxin und Digitoxin, sowie Sympathomimetika eingesetzt. Die Herzglycoside haben jedoch deutliche toxische Nebenwirkungen und eine geringe therapeutische Breite, während die Sympathomimetika unerwünschte chronotrope und arrythmogene Nebenwirkungen zeigen und oral nicht wirksam sind.

Diese Situation führte in den letzten Jahren zur Entwicklung neuer, positiv inotroper Substanzen, für die Amrinon (J.R. Benotti et al., N. Engl. J. Med. 299, 1373 (1978) und Milrinon (A.A. Alousi et al., J. Cardiovasc. Pharmacol. 5, 792 (1983) Beispiele sind.

Jedoch zeigen auch diese Substanzen ein unerwünschtes Spektrum von Nebenwirkungen bei oraler Anwendung, so daß ihre Anwendung auf andere Verabreichungsformen eingeschränkt wurde.

Der Erfindung liegt deshalb die Aufgabe zugrunde, neue wirksamere, positiv inotrope Substanzen mit einem besseren therapeutischen Profil zur Verfügung zu stellen.

Diese Aufgabe wird durch die Erfindung gelöst.

Gegenstand der Erfindung sind Dihydropyridazinon-Derivate der allgemeinen Formel I

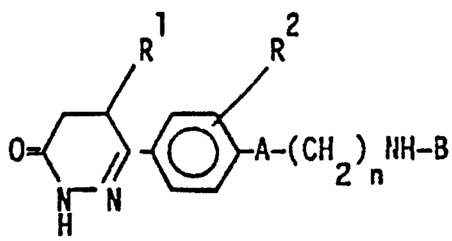

(I)

in der $R^1$ ein Wasserstoffatom oder eine gerad- oder verzweigtkettige $C_1$-$C_4$-Alkylgruppe bedeutet, $R^2$ für ein Wasserstoffatom, eine gerad- oder verzweigtkettige $C_1$-$C_4$-Alkylgruppe, eine $C_1$-$C_4$-Alkoxygruppe, ein Halogenatom, eine Nitrogruppe oder eine Aminogruppe steht, A für ein Sauerstoffatom, die Gruppe -NH- oder - $\underset{O}{\underset{\|}{C}}$ -NH-, einen in den Positionen 1 und 4 verknüpften Imidazolring oder eine Einfachbindung steht, B ein Wasserstoffatom oder die Gruppierung

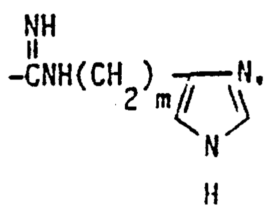

darstellt, in der m den Wert 2 oder 3 hat, und n eine ganze Zahl von 0 bis 6 ist, sowie die physiologisch annehmbaren Salze davon.

In der allgemeinen Formel I bedeutet $R^1$ ein Wasserstoffatom oder eine gerad- oder verzweigtkettige $C_1$-$C_4$-Alkylgruppe. Beispiele für solche gerad- oder verzweigtkettigen $C_1$-$C_4$-Alkylgruppen sind die Methyl-, Ethyl- und n-Propylgruppe, wobei die Methylgruppe bevorzugt wird. $R^2$ steht für ein Wasserstoffatom, eine gerad- oder verzweigtkettige $C_1$-$C_4$-Alkylgruppe, wie oben im Zusammenhang mit $R^1$ definiert, eine $C_1$-$C_4$-Alkoxygruppe, beispielsweise eine Methoxy- oder Ethoxygruppe, ein Halogenatom, beispielsweise ein Fluor-, Chlor- oder Bromatom, eine Nitrogruppe oder eine Aminogruppe.

A bedeutet ein Sauerstoffatom, die Gruppe -NH- oder - $\underset{O}{\underset{\|}{C}}$ -NH-, einen über die Positionen 1 und 4 verknüpften Imidazolring oder eine Einfachbindung. Die Gruppe - $\underset{O}{\underset{\|}{C}}$ -NH- kann dabei sowohl über die Carbonylgruppe als auch über das Stickstoffatom mit dem Phenylring verknüpft

sein, wobei die Verknüpfung über die Carbonylgruppe bevorzugt wird. Der Imidazolring ist vorzugsweise über das Stickstoffatom in Position 1 an den Phenylring gebunden. Die durch $-(CH_2)_n-$ angegebene Alkylengruppe ist über die Position 4 verknüpft.

In der allgemeinen Formel I steht weiterhin B für ein Wasserstoffatom oder die Gruppierung

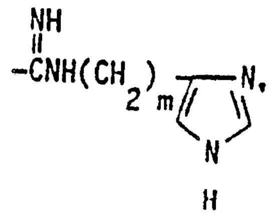

in der m den Wert 2 oder 3, vorzugsweise 3, hat. n ist eine ganze Zahl von 0 bis 6.

Eine bevorzugte Verbindungsgruppe gemäß der vorliegenden Erfindung ist dadurch gekennzeichnet, daß in der allgemeinen Formel I $R^1$ für ein Wasserstoffatom oder eine gerad- oder verzweigtkettige $C_1$-$C_4$-Alkylgruppe steht, $R^2$ ein Wasserstoffatom, eine gerad- oder verzweigtkettige $C_1$-$C_4$-Alkylgruppe, eine $C_1$-$C_4$ Alkoxygruppe, ein Halogenatom, eine Nitrogruppe oder eine Aminogruppe bedeutet, A für ein Sauerstoffatom, die Gruppe -NH- oder $-\underset{\overset{\|}{O}}{C}-NH-$, einen in den Positionen 1 und 4 verknüpften Imidazolring oder eine Einfachbindung steht, B ein Wasserstoffatom ist und n eine ganze Zahl von 0 bis 6 ist.

Eine weitere bevorzugte Verbindungsgruppe gemäß der vorliegenden Erfindung ist dadurch gekennzeichnet, daß in der allgemeinen Formel I $R^1$ ein Wasserstoffatom oder eine gerad- oder verzweigtkettige $C_1$-$C_4$-Alkylgruppe bedeutet, $R^2$ für ein Wasserstoffatom, eine gerad- oder verzweigtkettige $C_1$-$C_4$-Alkylgruppe, eine $C_1$-$C_4$-Alkoxygruppe, ein Halogenatom, eine Nitrogruppe oder eine Aminogruppe steht, A für ein Sauerstoffatom, die Gruppe -NH- oder $-\underset{\overset{\|}{O}}{C}-NH-$, einen in den Positionen 1 und 4 verknüpften Imidazolring oder eine Einfachbindung steht, B die Gruppierung

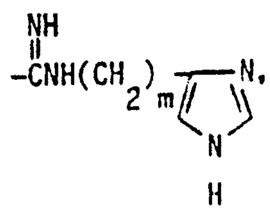

ist, in der m den Wert 2 oder 3 hat, und n eine ganze Zahl von 0 bis 6 ist.

Eine weitere bevorzugte Verbindungsgruppe gemäß der vorliegenden Erfindung ist dadurch gekennzeichnet, daß in der allgemeinen Formel I $R^1$ für eine gerad- oder verzweigtkettige $C_1$-$C_4$-Alkylgruppe, insbesondere für eine Methylgruppe, steht, $R^2$ ein Wasserstoffatom bedeutet, A für ein Sauerstoffatom, die Gruppe -NH- oder $-\underset{\overset{\|}{O}}{C}-NH-$, einen in den Positionen 1 und 4 gebundenen Imidazolring oder eine Einfachbindung steht, B ein Wasserstoffatom ist und n eine ganze Zahl zwischen 0 und 6 ist.

Schließlich ist eine weitere bevorzugte Verbindungsgruppe gemäß der vorliegenden Erfindung dadurch gekennzeichnet, daß in der allgemeinen Formel I $R^1$ für Wasserstoffatom oder eine gerad- oder verzweigtkettige $C_1$-$C_4$-Alkylgruppe, insbesondere eine Methylgruppe, steht und $R^2$ ein Wasserstoffatom bedeutet, A ein Sauerstoffatom, die Gruppe -NH- oder $-\underset{\overset{\|}{O}}{C}-NH-$, einen in den Positionen 1 und 4 verknüpften Imidazolring oder eine Einfachbindung darstellt, B die Gruppierung

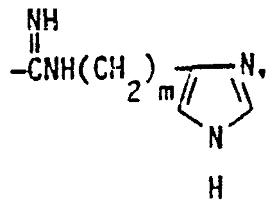

ist, in der m den Wert 2 oder 3, insbesondere 3, hat, und n eine ganze Zahl von 0 bis 6 ist.

Ein weiterer Gegenstand der vorliegenden Erfindung ist ein Verfahren zur Herstellung der oben beschriebenen Verbindungen, das dadurch gekennzeichnet ist, daß man

a) zur Herstellung von Verbindungen der allgemeinen Formel I, bei denen $R^1$, $R^2$, A und n wie oben definiert sind und B für ein Wasserstoffatom steht,

$a_1$) von einer Phthalimidverbindung der allgemeinen Formel II

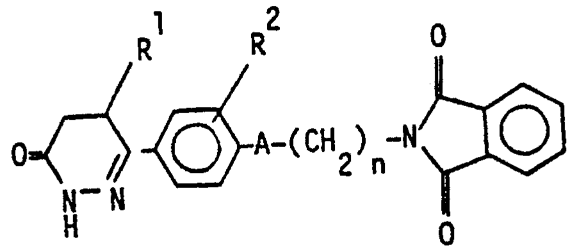

(II)

in der $R^1$ , $R^2$, A und n die oben angegebenen Bedeutungen besitzen, durch Umsetzung mit Hydrazin oder einem chemischen Äquivalent davon, einem aliphatischen primären Amin oder eine Säure die Phthalimidgruppe abspaltet und dadurch in eine Verbindung der allgemeinen Formel I überführt oder

$a_2$) eine Verbindung der allgemeinen Formel III

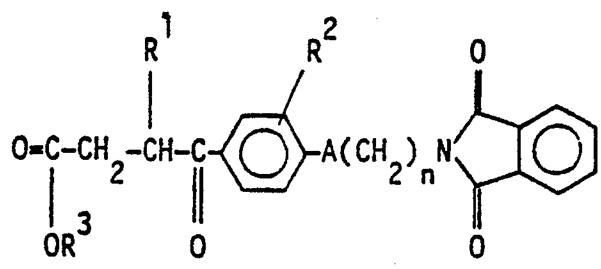

(III)

in der $R^1$, $R^2$, A und n die oben angegebenen Bedeutungen haben und $R^3$ für eine gegebenenfalls substituierte $C_1$-$C_4$-Alkylgruppe oder ein Wasserstoffatom steht, mit Hydrazin oder einem chemischen Äquivalent davon unter gleichzeitigem Ringschluß zum Pyridazinonring und Hydrazinolyse der Phthalimidschutzgruppe und unter Erhalt einer Verbindung der allgemeinen Formel I umsetzt oder

b) zur Herstellung von Verbindungen der allgemeinen Formel I, bei denen $R^1$, $R^2$, A und n wie oben definiert sind und B für die Gruppierung

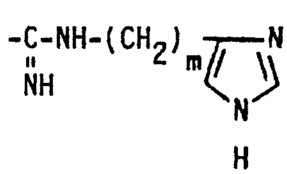

steht, in der m den Wert 2 oder 3 hat,

$b_1$) eine Verbindung der allgemeinen Formel IV

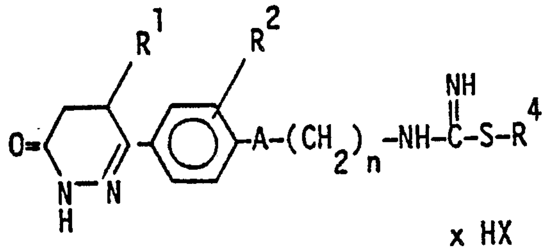

(IV)

in der $R^1$, $R^2$, A und n wie oben definiert sind, $R^4$ für eine gegebenenfalls substituierte $C_1$-$C_4$-Alkylgruppe steht und X ein Halogenatom oder die Gruppe $-OSO_2OR^4$ bedeutet, mit einem m-(Imidazol-4-yl)alkylamin der allgemeinen Formel V

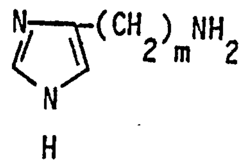

(V)

in der m den Wert 2 oder 3 hat, zu einer Verbindung der allgemeinen Formel I umsetzt oder

$b_2$) eine Verbindung der allgemeinen Formel VI

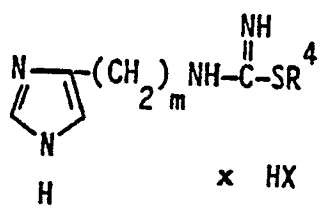

(VI)

in der m, $R^4$ und X wie oben definiert sind, mit einer Verbindung der allgemeinen Formel Ia

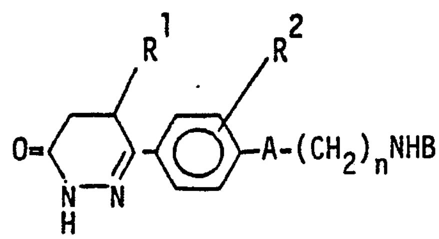

(Ia)

in die $R^1$, $R^2$, A und n die oben angegebenen Bedeutungen haben und B für ein Wasserstoffatom steht, zu einer Verbindung der allgemeinen Formel I umsetzt und daß man gegebenenfalls die nach $a_1$) bis $a_2$) oder $b_1$) bis $b_2$) erhaltene Verbindung in an sich bekannter Weise in ihr physiologisch annehmbares Salz umwandelt.

Somit können erfindungsgemäße Verbindungen der allgemeinen Formel I, bei denen $R^1$, $R^2$, A und n wie oben definiert sind und B für ein Wasserstoffatom steht, nach zwei verschiedenen Verfahrensvarianten hergestellt werden, nämlich

$a_1$) durch Umsetzung einer Verbindung der allgemeinen Formel II

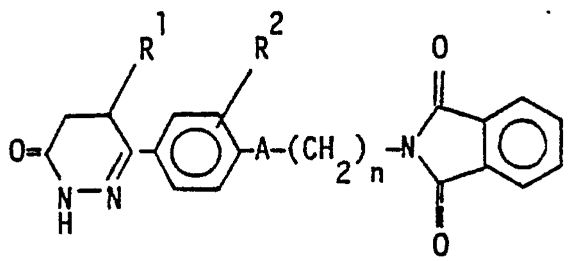

(II)

in der $R^1$, $R^2$, A und n die oben angegebenen Bedeutungen haben, mit Hydrazin bzw. einem chemischen Äquivalent davon, einem aliphatischen primären Amin, zum Beispiel Methylamin, oder einer Säure. Chemische Äquivalente des Hydrazins sind insbesondere Hydrazinhydrat, Hydrazinethanolat bzw. ähnliche Solvate oder dessen Salze. Hydrazin wird vorzugsweise in Form von Hydrazinhydrat eingesetzt. Die Umsetzung mit Hydrazin bzw. dessen Äquivalenten und Aminen erfolgt vorzugsweise mit einem Überschuß an Reagenz und in einem polaren Lösungsmittel, wie zum Beispiel einem Alkohol, wie Methanol, Ethanol oder Isopropanol. Die Reaktion wird bei Temperaturen von Raumtemperatur bis zum Siedepunkt des verwendeten Lösungsmittels, vorzugsweise bei Rückflußtemperatur durchgeführt. Bei der Verwendung von Aminen kann die Umsetzung gegebenenfalls unter erhöhtem Druck erfolgen. Die saure Hydrolyse der Verbindungen der allgemeinen Formel II wird in einer wäßrigen Mineralsäure, wie Chlor-, Brom- oder Jodwasserstoffsäure, Schwefelsäure oder Phosphorsäure, in einer verdünnten organischen Säure, wie zum Beispiel Essigsäure, bzw. in Gemischen von wäßrigen Mineralsäuren und organischen Säuren, bei erhöhter Temperatur, vorzugsweise bei Rückflußtemperatur, durchgeführt. Bei dieser Umsetzung wird von der Verbindung der allgemeinen Formel II die Phthalimidgruppe abgespalten, wodurch die Verbindung der allgemeinen Formel I erhalten wird.

Die weitere Möglichkeit ist

$a_2$) die Umsetzung einer Verbindung der allgemeinen Formel III

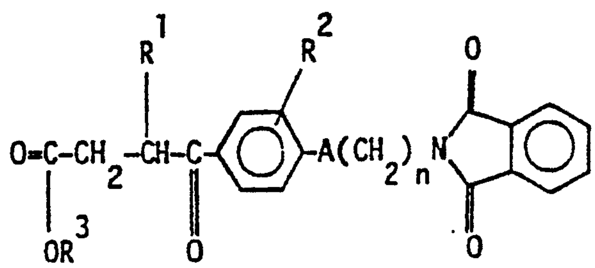

(III)

in der $R^1$, $R^2$, A und n wie oben definiert sind und $R^3$ eine gegebenenfalls substituierte $C_1$-$C_4$-Alkylgruppe, beispielsweise eine Methyl- oder Ethylgruppe, welche gegebenenfalls - beispielsweise durch ein Halogenatom, wie oben definiert, eine $C_1$-$C_4$-Alkylgruppe, wie oben definiert, oder eine $C_1$-$C_4$-Alkoxygruppe, wie oben definiert - substituiert sein kann, oder ein Wasserstoffatom ist, mit Hydrazin oder seinem chemischen Äquivalent, wie oben im Zusammenhang mit der Verfahrensvariante $a_1$) definiert. Die Umsetzung erfolgt wie im Fall der Verfahrensvariante $a_1$) vorzugsweise mit einem Überschuß an Hydrazin bzw. seinem chemischen Äquivalent. Auch in diesem Falle wird das Hydrazin vorzugsweise in Form von Hydrazinhydrat eingesetzt. Die Umsetzung erfolgt ebenfalls in einem polaren Lösungsmittel, beispielsweise einem Alkohol, wie Methanol, Ethanol, Isopropanol oder n-Butanol. Die Reaktion wird bei erhöhter Temperatur, vorzugsweise Rückflußtemperatur des verwendeten Lösungsmittels, und gegebenenfalls mit Säurekatalyse, zum Beispiel mit Essigsäure, durchgeführt. Bei der Umsetzung erfolgt gleichzeitig Ringschluß zum Pyridazinonring und die Hydrazinolyse der Phthalimidschutzgruppe, wodurch eine Verbindung der allgemeinen Formel I erhalten wird.

Erfindungsgemäße Verbindungen der allgemeinen Formel I, bei denen $R^1$, $R^2$, A und n wie oben angegebenen definiert sind und B für die Gruppierung

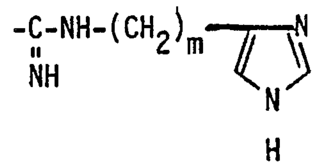

steht, werden nach einer der folgenden Verfahrensvarianten hergestellt:

$b_1$) durch Umsetzung eines Isothiuroniumsalzes der allgemeinen Formel IV

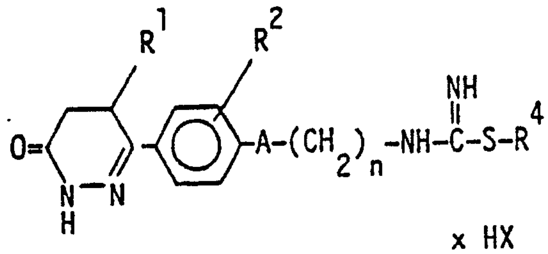

x HX

(IV)

in der $R^1$, $R^2$, A und n wie oben definiert sind, $R^4$ eine $C_1$-$C_4$-Alkylgruppe - beispielsweise eine Methyl- oder Ethylgruppe, die gegebenenfalls beispielsweise durch ein Halogenatom, wie oben definiert, substituiert sein kann - bedeutet und X für ein Halogenatom, beispielsweise ein Fluor-, Chlor- oder Bromatom, oder die Gruppe $-OSO_2OR^4$ steht, mit einem m-(Imidazol-4-yl)alkylamin der allgemeinen Formel V

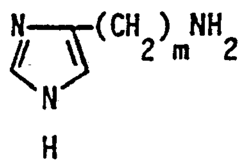

(V)

in der m den Wert 2 oder 3 hat. Die Umsetzung der Reaktanten erfolgt in äquimolaren Mengen und in einem polaren Lösungsmittels, wie zum Beispiel Pyridin, Acetonitril oder einem Alkohol, wie Ethanol, Isopropanol oder n-Butanol, bei Temperaturen von Raumtemperatur bis zur Rückflußtemperatur des verwendeten Lösungsmittels, vorzugsweise bei Rückflußtemperatur;

oder

$b_2$) durch Umsetzung einer Verbindung der allgemeinen Formel VI

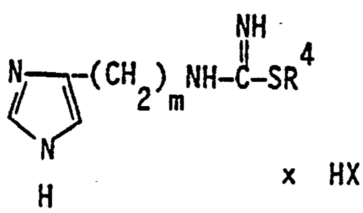

x HX

(VI)

(VI)

in der $R^4$, m und X die oben genannten Bedeutungen haben, mit einem Amin der allgemeinen Formel Ia

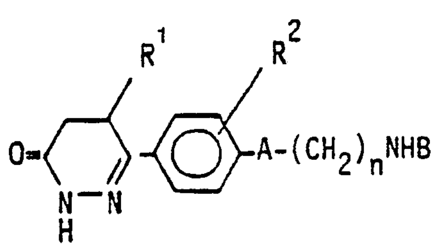

(Ia)

in der $R^1$, $R^2$, A und n wie oben definiert sind und B für ein Wasserstoffatom steht.

Bezüglich der verwendeten Lösungsmittel, der Reaktionstemperatur und der Stöchiometrie der Reaktionspartner gilt für diese Umsetzungen das im Zusammenhang mit der Verfahrensvariante $b_1$) Gesagte.

Die nach den einzelnen Verfahrensvarianten erhaltenen erfindungsgemäßen Verbindungen werden in üblicher Weise isoliert und gereinigt, beispielsweise durch Kristallisation aus geeigneten Lösungsmitteln, chromatographische Arbeitsweisen etc.

Die erfindungsgemäßen Verbindungen der allgemeinen Formel I können sowohl in mehreren stereoisomeren Formen als auch in mehreren tautomeren Formen vorliegen. Die Erfindung umfaßt daher auch alle stereoisomeren als auch tautomeren Formen sowie die Hydrate der Verbindungen der allgemeinen Formel I.

Die in den einzelnen Verfahrensvarianten erhaltenen Verbindungen können gegebenenfalls in ihr physiologisch annehmbares Salz umgewandelt werden. Diese Salze können zum Beipsiel mit Mineralsäuren, wie Chlor-, Brom- oder Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure, Embonsäure etc., gebildet werden.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung eines oder mehrerer pharmazeutischer Träger oder Verdünnungsmittel hergestellt werden.

Die erfindungsgemäßen Verbindungen können daher für die orale, bukkale, topische, parenterale oder rektale Verabreichung formuliert werden.

Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind.

Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/ oder Dispergierungsmittel, enthalten.

Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, je nach Zustand des Patienten. Im Einzelfall kann es gegebenenfalls er forderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß.

Die folgenden pharmakologischen Daten zeigen die in verschiedenen Screening-Tests erhaltenen Wirkungsdaten erfindungsgemäßer Verbindungen.

1.) Bestimmung der positiv inotropen Wirkung am isolierten, elektrisch gereizten Meerschweinchen-Papillarmuskel

a) Methode

Für die Untersuchungen wurden elektrisch gereizte Papillarmuskelpräparate aus der rechten Herzkammer von Meerschweinchen benutzt. Das Gewicht der Tiere lag zwischen 300 und 500 g. Die Tiere wurden durch Nackenschlag getötet und entblutet. Das Herz wurde schnell entnommen; die weitere Präparation erfolgte in zimmerwarmer Krebs-Lösung. Die isolierten Präparate wurden dann an Plexiglashaltern mit Reizelektroden befestigt und in Badgefäße von 100 ml Inhalt gebracht. Die Begasung erfolgte mit Carbogen (95 % $O_2$ + 5 % $CO_2$) bei einer Versuchstemperatur von 31° C.

Die Kontraktionen wurden über elektromechanische Wandler (Statham UC 2) aufgenommen und über Gleichspannungsmeßbrücken (H. Sachs-Elektronik) auf einen Direktschreiber registriert. Gleichzeitig wurden die Signale elektronisch ausgewertet und digital ausgedruckt (DIO-1, Fa. mikro-mess). Die Messungen erfolgten immer dann, wenn sich ein "steady-state" ausgebildet hatte.

Die Vorspannung der Papillarmuskel betrug 0,5 g. Die Reizfrequenz war 1 Hz, die Dauer der Rechteckimpulse 3 ms. Die Stimulation erfolgte mit überschwelligen Reizstromstärken.

b) Meßwerte

| Beispiel Nr. | max. Kontraktionskraftzunahme in % | $pD_2$-Wert |
|---|---|---|
| 1 | + 18 % | 5.6 |
| 2 | + 81 % | 5.3 |
| 3 | + 31 % | 6.8 |
| 6 | + 48 % | 5.2 |
| 7 | + 51 % | 4.9 |
| 8 | + 43 % | 6.2 |
| 9 | + 66 % | 6.2 |
| Amrinon (Vergleich) | + 34 % | 5.8 |

2.) Untersuchungen am isolierten, perfundierten Langendorff-Herzen (Meerschweinchen)

a) Methode

Zur Bestimmung der hämodynamischen Effekte der erfindungsgemäßen Verbindungen an isolierten, perfundierten Meerschweinchenherzen wurde die Anordnung von Langendorff nach P.R. Beckett (J. Pharm. Pharmacol. 22, 818 (1970)) und R.M. Abel u. R.L. Reis (Circ. Res. 27, 961 (1970)) modifiziert. Die spontan schlagenden Meerschweinchenherzen waren linksventrikulär katheteri siert und wurden mit Lösungen der Prüfsubstanzen in physiol. Kochsalzlösung / Ethanol (9:1) in Konzentrationen von $10^{-5}$ bzw. $10^{-6}$ Mol/l mit einem konstanten Perfusionsdruck von 60 mm Hg perfundiert.

b) Meßwerte

| Beispiel Nr. | Konz. (Mol/l) | Prozentuale Veränderungen gegenüber den Kontrollen | | |
|---|---|---|---|---|
| | | Kontraktilität dp/dt | Koronarfluß | Herzfrequenz |
| 2 | $10^{-6}$ | + 46 % | + 25 % | + 9 % |
| 3 | $10^{-5}$ | + 73 % | + 5 % | + 5 % |
| 6 | $10^{-5}$ | + 163 % | + 129 % | + 15 % |
| 7 | $10^{-6}$ | + 190 % | + 23 % | + 25 % |
| 8 | $10^{-6}$ | + 130 % | + 52 % | + 23 % |
| Amrinon (Vergleich) | $10^{-5}$ | + 25 % | + 46 % | + 18 % |

Beispiel 1

6-[4-[4-(3-Aminopropyl)-1H-imidazol-1-yl]phenyl]-4,5-dihydro-3(2H)-pyridazinon-dihydrochlorid

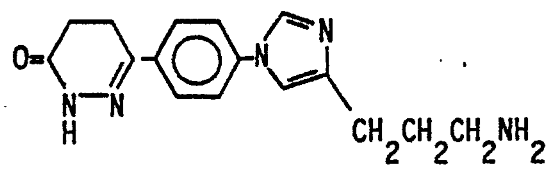

x 2 HCl

a) 3-[4-[4-(3-Phthalimidopropyl)-1H-imidazol-1-yl]benzoyl]-propionsäure

Zu einer Suspension von 1,20 g (40 mmol) Natriumhydrid (80 % auf Paraffinöl) in 50 ml Dimethylsulfoxid werden portionsweise 5,10 g (20 mmol) 4-(3-Phthalimidopropyl)-imidazol und dann 3,92 g (20 mmol) 3-(4-Fluorbenzoyl)-propionsäure gegeben. Das Reaktionsgemisch wird zunächst bis zur beendeten Gasentwicklung bei Raumtemperatur und dann 17 h bei 100° C gerührt. Nach Abkühlen wird die Mischung auf 50 ml Wasser gegossen und mit 3 x 50 ml Dichlormethan extrahiert. Die wässrige Phase wird dann mit 2 N Salzsäure auf pH 5 gestellt, der ausgefallene Feststoff abgesaugt und aus Methanol umkristallisiert.

Ausbeute: 4,92 g (57 %)
Schmp. 200 - 203° C
$C_{24}H_{21}N_3O_5$ (431.45)

b) 6-[4-[4-(3-Aminopropyl)-1H-imidazol-1 yl]phenyl]-4,5-dihydro-3(2H)-pyridazinon-dihydrochlorid

3.02 g (7 mmol) 3-[4-[4-(3-Phthalimidopropyl)-1H-imidazol-1-yl]benzoyl] -propionsäure und 1,7 ml (35 mmol) Hydrazinhydrat werden in 30 ml Ethanol 6 h gekocht. Nach Abkühlen auf Raumtemperatur wird der erhaltene Feststoff abgesaugt und die Mutterlauge i. Vak. vollständig eingeengt. Der Rückstand wird in 10 ml Ethanol aufgenommen und mit 2 ml isopropanolischer Salzsäure (~10 Mol/l) versetzt. Der ausgefallene Feststoff wird abgesaugt, mit etwas Methanol gewaschen und i. Vak. getrocknet.

Ausbeute: 1,22 g (47 %)
Schmp. 235 - 238° C (Zers.)
$C_{16}N_{21}Cl_2N_5O$ (370.28)

$^1$H-NMR Daten ($D_2O$, TMSP als interner Standard)
δ = 2,2 (quin) 2 H,
2,6 (t) 2 H,
2,9 - 3,4 (m) 6 H,
7,7 - 8,1 (m) 5 H,
9,15 (d) 1 H ppm.

Beispiel 2

6-[4-[4 (3-Aminopropyl)-1H-imidazol-1-yl]phenyl]-4,5-dihydro-5-methyl-3(2H)-pyridazinon-dihydrochlorid

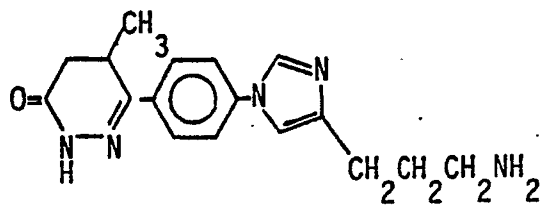

x 2 HCl

a) 3-[4-[4-(3-Phthalimidopropyl)-1H-imidazol-1-yl]benzoyl]-buttersäure

Hergestellt analog zu Beispiel 1 a) aus 2,6 g (10,2 mmol) 4-(3 Phthalimidopropyl)-imidazol und 2,2 g (10,5 mmol) 3-(4-Fluorbenzoyl)-buttersäure. Man erhält 3,4 g (75 %) eines bräunlichen, zähen Öls, das ohne Reinigung in die nächste Stufe eingesetzt wird.

$C_{25}H_{23}N_3O_5$ (445,50)

b) 6-[4-[4-(3-Aminopropyl)-1H-imidazol-1-yl]phenyl]-4,5 dihydro-5-methyl-3(2H)-pyridazinon-dihydrochlorid

2,3 g (5,2 mmol) 3-[4-[4-(3-Phthalimidopropyl)-1H-imidazol-1-yl]benzoyl] -buttersäure und 1,25 ml (25,5 mmol) Hydrazinhydrat werden in 20 ml Ethanol gekocht. Nach 6 h wird das Reaktionsgemisch abgekühlt, der erhaltene Feststoff abgesaugt und das Filtrat i. Vak. vollständig eingeengt. Der verbleibende Rückstand wird in 10 ml Ethanol gelöst und unter Rühren mit 1,5 ml isopropanolischer Salzsäure versetzt. Beim Nachrühren bei Raumtemperatur kristallisiert ein farbloser Feststoff, der abgesaugt, mit Ethanol gewaschen und i.Vak. getrocknet wird.

Ausbeute: 1,03 g (52 %)
Schmp. 175° C
$C_{17}H_{22}Cl_2N_5O$ (383,30)
$^1$H-NMR-Daten ($D_2O$, TMSP als interner Standard)
δ = 1,1 (d) 3 H,
2,2 (quin) 2 H,
2,5 - 3,0 (m) 4 H,
3,1 - 3,6 (m) 3 H,
7,6 - 8,0 (m) 5 H,
8,7 (d) 1 H ppm.

Beispiel 3

6-[4-(3-Aminopropylamino)phenyl]-4,5-dihydro-5-methyl-3(2H)-pyridazinon

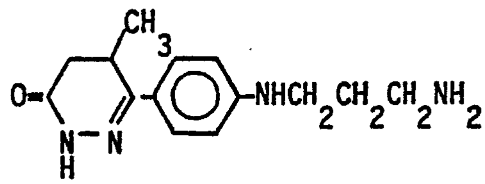

a) 3-[4-[N-(3-Phthalimidopropyl)acetamido]benzoyl]buttersäure

30,0 g (120 mmol) 3-(4-Acetamidobenzoyl)-buttersäure werden zu einer Suspension von 7,6 g (253 mmol) Natriumhydrid (80 %) in 150 ml Dimethylsulfoxid gegeben und die Mischung 30 Minuten bei 60° C gerührt. Nach Zugabe von 33,2 g (120 mmol) N-(3 Brompropyl)-phthalimid wird 6 h bei 80° C weitergerührt. Das Reaktionsgemisch wird nach Abkühlen auf 200 ml Eiswasser gegeben und mit 3 x 100 ml Dichlormethan extrahiert. Die Extrakte werden verworfen. Die wässrige Phase wird dann mit 2 N Salzsäure auf pH 6,5 eingestellt und abermals mit 3 x 100 ml Dichlormethan extrahiert. Die vereinigten organischen Phasen werden getrocknet und im Vakuum eingedampft. Das verbleibende Öl kristallisiert beim Verreiben mit 20 ml Ethanol.

Ausbeute: 23,1 g (44 %)
$C_{24}H_{24}N_2O_6$ (436,46)

b) 6-[4-[N-(3-Aminopropyl)acetamido]phenyl]-4,5-dihydro-5-methyl-3(2H)-pyridazinon-hydrochlorid

4,7 g (10,8 mmol) 3-[4-[N-(3-Phthalimidopropyl)acetamido]benzoyl]-buttersäure und 2,7 ml (55 mmol) Hydrazinhydrat werden in 60 ml Ethanol 1 h unter Rückfluß gekocht. Das nach Absaugen des ausgefallenen Feststoffs erhaltene Filtrat wird i. Vak. eingedampft und der Rückstand mit 20 ml 2 N Salzsäure aufgenommen. Nach erneuter Filtration wird die Mutterlauge i. Vak. eingedampft. Es verbleiben 2,82 g (80 %) eines orangegelben, zähen Öls, das ohne Reinigung weiter umgesetzt wird.

$C_{16}H_{23}ClN_4O_2$ (338,84)

c) 6-[4-(3-Aminopropylamino)phenyl]-4,5-dihydro-5-methyl-3(2H)-pyridazinon

2,8 g (8,3 mmol) 6-[4-[N-(3-Aminopropyl)acetamido]pheny]-4,5-dihydro-5-methyl-3(2H)-pyridazinon hydrochlorid werden in 30 ml 2N Salzsäure 2 h unter Rückfluß gekocht. Die Lösung wird i. Vak. eingedampft und der Rückstand in 20 ml gesättigtem Kaliumcarbonat aufgenommen. Nach zweimaliger Extraktion mit jeweils 30 ml Isopropanol werden die vereinigten organischen Phasen mit Kaliumcarbonat getrocknet und i. Vak. eingedampft. Der Rückstand wird mit Ethylacetat/Ethanol/konz. Ammoniak (15:10:2) an Kieselgel chromatographisch gereinigt.

Ausbeute: 1,2 g (56 %)
Farbloser Feststoff vom Schmp. 68 - 70° C
$C_{14}H_{20}N_4O$ (260.34)
$^1$H-NMR-Daten ($CD_3OD$, TMS als interner Standard)

$\delta$ = 1,1 (d) 3 H,
1,7 (quin) 2 H,
2,2 - 2,9 (m) 4 H,
3,1 - 3,5 (m) 3 H,
4,9 (breit) 4 H, austauschbar mit $D_2O$,
6,7 (d) 2 H,
7,7 (d) 2 H ppm.

Beispiel 4

6-[4-(3-Aminopropoxy)phenyl]-4,5-dihydro-5-methyl-3(2H)pyridazinon

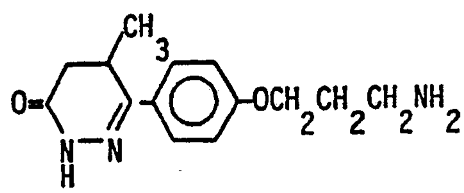

a) 3-[4-(3-Phthalimidopropoxy)benzoyl]-buttersäuremethylester

26,0 g (200 mmol) wasserfreies Aluminiumchlorid werden in 200 ml 1,2-Dichlorethan suspendiert und auf 10° C abgekühlt. Unter weiterem Kühlen werden 8,2 g (50 mmol) 3-Methoxycarbonyl-2-methyl-propionsäurechlorid in 20 ml 1,2-Dichlorethan und dann 14.0 g (50 mmol) (3-Phthalimidopropyl)-phenylether in 50 ml 1,2-Dichlorethan so zugetropft.,daß die Innentemperatur nicht über 10° C ansteigt.

Nach 18-stündigem Nachrühren bei Raumtemperatur wird die Mischung auf 400 ml Eiswasser gegossen. Die wässrige Phase wird nach Phasentrennung noch zweimal mit 100 ml Dichlormethan extrahiert. Die vereinigten organischen Phasen werden mit Natriumsulfat getrocknet, filtriert und i. Vak. eingedampft. Man erhält 20,9 g (quant.) eines organgegelben Öls, das im Eisbad nach Versetzen mit 50 ml Ethanol erstarrt. Das Produkt wird ohne Reinigung weiter umgesetzt.

$C_{23}H_{23}NO_6$ (409,44)

b) 6-[4-(3-Aminopropoxy)phenyl]-4,5-dihydro-5-methyl-3(2H)-pyridazinon

20,5 g ( 50 mmol) 3-[4-(3-Phthalimidopropoxy)benzoyl]-buttersäuremethylester werden in 120 ml Ethanol mit 12 ml (250 mmol) Hydrazinhydrat und 0,1 ml Eisessig versetzt und 1,5 h unter Rückfluß gekocht. Die erhaltene Suspension wird nach Abkühlen i.Vak. eingedampft und der Rückstand in 300 ml 2N Salzsäure aufgenommen. Nach Abfiltrieren des Niederschlags wird das Filtrat mit konz. Ammoniak auf pH 10 gestellt und mit 3 x 100 ml Dichlormethan extrahiert. Die vereinigten organischen Phasen werden getrocknet, filtriert und im Vakuum eingedampft. Das verbleibende Öl kristallisiert beim Verrühren mit 30 ml Ethylacetat.

Ausbeute: 7,9 g (61 %)
Schmp. 119 - 121° C
$C_{14}H_{19}N_3O_2$ (261,33)
$^1H$-NMR-Daten ($d_6$-DMSO, TMS als interner Standard)
$\delta$ = 1,0 - 1,2 (2 d) 3 H,

1,5 - 2,0 (m) 4 H, 2 H austauschbar mit $D_2O$
2,1 - 3,5 (m) 3 H,
2,7 (t) 2 H,
4,1 (t) 2 H,
7,0 - 7,2 (dd) 2 H,
7,7 - 7,9 (dd) 2 H,
11,0 (breit) 1 H, austauschbar mit $D_2O$

Beispiel 5

6-[4-(3-Aminopropyl)phenyl]-4,5-dihydro-5-methyl-3(2H)-pyridazinon

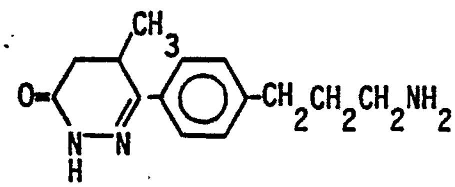

a) 3-[4-(3-Phthalimidopropyl)benzoyl]-buttersäuremethylester

8,2 g (50 mmol) 3-Methoxycarbonyl-2-methyl-propionsäurechlorid und 13,3 g (50 mmol) 3-Phthalimidopropyl-benzol werden analog zu Beispiel 4 a) mit 26 g Aluminiumchlorid in 1,2 Dichlorethan umgesetzt. Das erhaltene Öl kristallisiert aus Ethanol in beigefarbenen Kristallen.

Ausbeute 17,3 g (88 %)
Schmp. 96 - 97° C
$C_{23}H_{23}NO_5$ (393,44)

b) 6-[4-(3-Aminopropyl)phenyl]-4,5-dihydro-5-methyl-3(2H)-pyridazinon

Analog zu Beispiel 4 b) erhält man aus 17,3 g (44 mmol) 3-[4-(3-Phthalimido-propyl)benzoyl]-buttersäuremethylester und 11 ml (226 mmol) Hydrazinhydrat 9,3 g eines gelblichen Rohprodukts, das aus Ethylacetat umkristallisiert wird.

Ausbeute 6,0 g (54 %)
Schmp. 115 - 117° C
$C_{14}H_{19}N_3O$ (245,32)

$^1H$ NMR Daten ($d_6$-DMSO, TMS als interner Standard)
$\delta$ = 1,15 (d) 3 H,
1,4 - 2,0 (m) 4 H, 2 H austauschbar mit $D_2O$
2,3 - 2,8 (m) 6 H,
2,9 - 3,3 (m) 1 H,
7,3 - 7,5 (m) 2 H,
7,7 - 7,9 (m) 2 H,
10,0 (breit) 1 H, austauschbar mit $D_2O$ ppm.

Beispiel 6

$N^1$-[4-(4-Methyl-6-oxo-1,4,5,6-tetrahydropyridazin-3-yl)phenyl]-$N^2$-[ 3-(1H imidazol-4-yl)propyl]-guanidin-hydrojodid

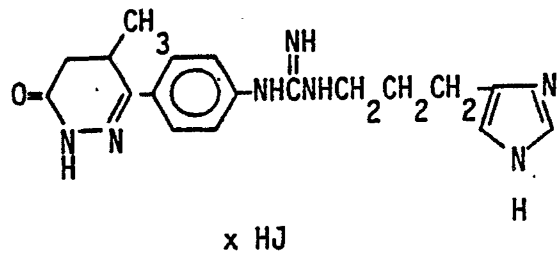

x HJ

a) $N^1$-Benzoyl-$N^2$-[4-(4-methyl-6-oxo-1,4,5,6-tetrahydropyridazin-3-yl)phenyl]-thioharnstoff

Zu einer Suspension von 6,00 g (29,5 mmol) 6-(4-Aminophenyl)-5-methyl-4,5-dihydro-3(2H)-pyridazinon in 70 ml Dichlormethan werden bei Raumtemperatur 4,85 g (29,7 mmol) Benzoylisothiocyanat in 20 ml Dichlormethan getropft. Nach einstündigem Nachrühren bei Raumtemperatur wird der gebildete Niederschlag abgesaugt und mit 20 ml Dichlormethan gewaschen. Man erhält 10,21 g (94 %) blaßgelbe Kristalle.

Schmp. 235 - 237° C
$C_{19}H_{18}N_4O_2S$ (366,44)

b) N-[4-(4-Methyl-6-oxo-1,4,5,6-tetrahydropyridazin-3-yl)phenyl]-thioharnstoff

10,17 g (27,8 mmol) $N^1$-Benzoyl-$N^2$-[4 (4-methyl-6-oxo-1,4,5,6-tetrahydropyridazin-3-yl)phenyl]-thioharnstoff werden in 300 ml Methanol und 95 ml Wasser mit 7,6 g (55 mmol) Kaliumcarbonat 1 h gekocht. Nach Abkühlen auf Raum-temperatur unter Rühren werden die entstandenen Kristalle abgesaugt, mit 20 ml Methanol gewaschen und i. Vak. getrocknet.

Ausbeute : 6,4 g (88%)
Farblose Kristalle vom Schmp. 233 - 234° C
$C_{12}H_{14}N_4OS$ (262,33)

c) N-[4-(4-Methyl-6-oxo-1,4,5,6-tetrahydropyridazin-3-yl)phenyl]-S-methylisothiuroniumjodid

6,4 g (24,4 mmol) N-[4-(4-Methyl-6-oxo-1,4,5,6-tetrahydropyridazin-3-yl)phenyl] -thioharnstoff werden in 150 ml Ethanol suspendiert und mit 2,0 ml (32 mmol) Methyljodid versetzt. Nach zwanzigstündigem Rühren bei Raumtemperatur wird die nicht umgesetzte Ausgangsverbindung abgesaugt und die Mutterlauge i. Vak. eingedampft. Der feste Rückstand wird aus Ethanol/Methanol (4:1) umkristallisiert.

Ausbeute: 2,1 g (21 %)
Farblose Kristalle vom Schmp. 210° C
$C_{13}H_{17}JN_4OS$ (404,26)

d) $N^1$-[4-(4-Methyl-6-oxo-1,4,5,6-tetrahydropyridazin-3-yl)phenyl]-$N^2$-[3-(1H-imidazol-4-yl)propyl ]-guanidinhydrojodid

1,00 g (2,47 mmol) N-[4-(4-Methyl-6-oxo-1,4,5,6-tetrahydropyridazin-3-yl)phenyl] -S-methyl-isothiuroniumjodid und 0,31 g (2,5 mmol) 3-(1H-Imidazol-4-yl)propylamin werden in 50 ml Acetonitril unter Stickstoffatmosphäre 3 h gekocht. Nach Abdampfen des Lösungsmittels i. Vak. wird das erhaltene Öl an Kieselgel mit Dichlormethan/Methanol (90:10→80:20→50:50) als Laufmittel chromatographisch gereinigt. Die polare Hauptfraktion ergibt nach Eindampfen i. Vak. 0,73 g (61 %) eines farblosen, amorphen Feststoffs.

$C_{18}H_{24}JN_7O$ (481,33)
$^1$H-NMR-Daten ($d_6$-DMSO, TMS als interner Standard)
$\delta$ = 1,1 (d) 3 H,
1,8 (quin) 2H,
2,1 - 2,9 (m) 4 H,
3,2 - 3,6 (m) 3 H,
6,9 (s) 1 H,
7,3 (d) 2 H,
7,6 (s) 1 H,
7,9 (d) 2 H ppm.

Beispiel 7

$N^1$-[3-[N-[4-(4 Methyl-6-oxo-1,4,5,6-tetrahydropyridazin-3-yl)phenyl] carboxamido]propyl]-$N^2$- [3-(1H-imidazol-4-yl)propyl]-guanidin

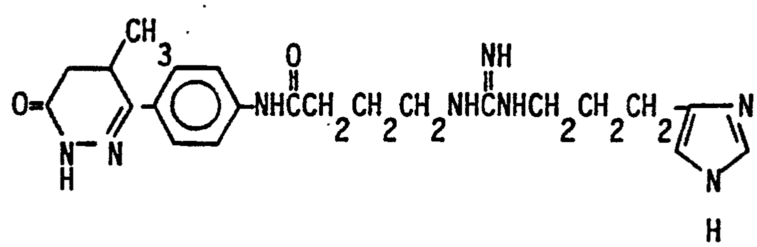

a) $N^1$-Benzoyl-$N^2$-[3-[N-[4-(4-methyl-6-oxo-1,4,5,6-tetrahydropyridazin-3-yl)phenyl carboxamido]propyl]-thioharnstoff

3,4 g (20,8 mmol) Benzoylisothiocyanat in 20 ml Ethylacetat werden während 15 Min. zu einer Suspension von 6,0 g (20,8 mmol) 4-Amino-N-[4-(4-methyl-6-oxo-1,4,5,6-tetrahydropyridazin-3-yl)phenyl] butyramid in 100 ml Acetonitril getropft. Nach einstündigem Nachrühren bei Raumtemperatur wird die klare Lösung i. Vak. eingedampft und der Rückstand mit einem Gemisch von 200 ml Methanol und ml Wasser verrührt. Dabei kristallisieren 3,7 g (40 %) eines farblosen Feststoffs.

Schmp. 190 - 192° C
$C_{23}H_{25}N_5O_3S$ (451,54)

b) N-[3-[N-(4-Methyl-6-oxo-1,4,5,6-tetrahydropyridazin-3-yl)phenyl] carboxamido]propyl]-thioharnstoff

Hergestellt analog zu Beispiel 6 b) aus 3,70 g (8,2 mmol) $N^1$-Benzoyl-$N^2$-[3-[N-[4-(4-methyl-6-oxo-1,4,5,6-tetrahydropyridazin-3-yl)phenyl] carboxamido]propyl]-thioharnstoff und 2,26 g (16,4 mmol) Kaliumcarbonat.

Ausbeute: 2,69 g (95 %)
Farblose Kristalle vom Schmp. 142 - 144° C
$C_{16}H_{21}N_5O_2S$ (347,44)

c) N-[3-[N-[4-(4-Methyl-6-oxo-1,4,5,6-tetrahydropyridazin-3-yl)phenyl] carboxamido]propyl]-S-methyl-isothiuroniumjodid

1,20 g (3,5 mmol) N-[3-[N-[4-(4-Methyl-6-oxo-1,4,5,6-tetrahydropyridazin-3-yl) phenyl]carboxamido]-propyl]-thioharnstoff und 0,30 ml (4,8 mmol) Methyljodid werden in 100 ml Ethanol 4 Tage bei Raumtemperatur gerührt. Der entstandene Feststoff wird abgesaugt, mit Ethanol gewaschen und i.Vak. getrocknet.

Ausbeute: 1,06 g (63 %)
Farblose Kristalle vom Schmp. 214 - 216° C
$C_{17}H_{24}JN_5O_2S$ (489,37)

d) $N^1$-[3-[N-[4-(4-Methyl-6-oxo-1,4,5,6-tetrahydropyridazin-3-yl)phenyl] carboxamido]propyl]-$N^2$-[3-(1H-imidazol-4-yl)propyl]-guanidin

1,00 g (2,04 mmol) N-[3-[N-[4-(4-Methyl-6-oxo-1,4,5,6-tetrahydropyridazin-3-yl)phenyl]carboxamido]-propyl]-S-methyl-isothiuroniumjodid und 0,26 g (2,06 mmol) 3-(1H-Imidazol-4-yl)-propylamin werden in 40 ml Acetonitril 4 Stunden gekocht. Die Lösung wird noch heiß von harzigen Verunreinigungen abdekantiert und dann i. Vak. eingedampft. Der Rückstand wird an Kieselgel mit Ethylacetat /Methanol/ konz. Ammoniak gesättigt mit Ammoniumchlorid (50:48:2) als Laufmittel chromatographiert. Die polare Hauptfraktion wird i.Vak. eingedampft, der Rückstand in 10 ml gesättigter Kaliumcarbonatlösung aufgenommen und mit 2 x 20 ml Isopropanol extrahiert. Die organ. Phase wird mit Kaliumcarbonat getrocknet, filtriert und i.Vak. zur Trockne eingedampft. Es verbleiben 0,31 g (35 %) eines farblosen, amorphen Feststoffs.

$C_{22}H_{30}N_8O_2$ (438,53)
$^1$H-NMR-Daten ($CD_3OD$, TMS als interner Standard)
$\delta$ = 1,15 (d) 3 H,
1,7 - 2,1 (m) 4 H,
2,3 - 2,8 (m) 6 H,
3,1 - 3,5 (m) 5 H,
5,0 (breit) 6 H, austauschbar mit $D_2O$
6,9 (s) 1 H,
7,7 (s) 1 H,
7,7 - 8,0 (m) 4 H ppm.

Beispiel 8

N¹-[3-[N-[4-(4-Methyl-6-oxo-1,4,5,6-tetrahydropyridazin-3-yl)phenyl]amino] propyl]-N²-[3-(1H-imidazol-4-yl)-propyl]-guanidin-dihydrobromid

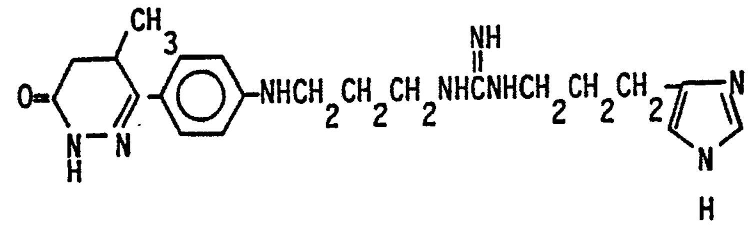

x 2 HBr

0,37 g (1,4 mmol) 6-[4-(3-Aminopropylamino)phenyl]-4,5-dihydro-5-methyl-3(2H)-pyridazinon und 0,51 g (1,4 mmol) N-[3-(1H-Imidazol-4-yl)propyl]-S-methyl-isothiuronium-dihydrobromid werden in 30 ml Acetonitril und 2 ml Pyridin 5 h unter Rückfluß gekocht. Die Lösung wird i. Vak. vollständig eingeengt und der Rückstand in 20 ml Ethanol verrührt. Unumgesetztes Isothiuroniumsalz wird abfiltriert und das Filtrat i. Vak. eingedampft. Das Rohprodukt wird an Kieselgel mit Ethylacetat/Ethanol/konz. Ammoniak (15:10:2) chromatographiert. Die Hauptfraktion ergibt nach Abdampfen des Laufmittels i. Vak. 0,16 g (36 %) eines gelblichen, amorphen Feststoffs.

$C_{21}H_{32}Br_2N_8O$ (572,36)
$^1$H-NMR-Daten ($CD_3OD$, TMS als interner Standard)
$\delta$ = 1,15 (d) 3 H,
1,8 - 2,2 (m) 4 H,
2,2 - 3,5 (m) 11 H,
4,9 (breit) 8 H, austauschbar mit $D_2O$,
6,7 - 6,9 (m) 2 H,
7,5 (s) 1 H,
7,7 - 7,9 (m) 2 H,
8,9 (d) 1 H ppm.

Beispiel 9

N¹-[3-[1-[4-(6-Oxo-1,4,5,6-tetrahydropyridazin-3-yl)phenyl]imidazol-4-yl] propyl]-N²-[3-(1H-imidazol-4-yl)-propyl]-guanidin

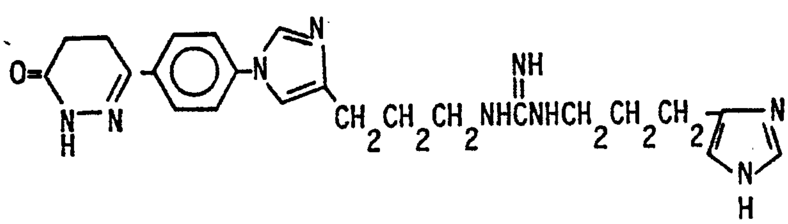

0,30 g (1,00 mmol) 6-[4-[4-(3-Aminopropyl)-1H-imidazol-1-yl]phenyl]-4,5 dihydro -3(2H)-pyridazinon und 0,37 g (1,02 mmol) N-[3-(1H-Imidazol-4-yl)propyl] -S-methyl-isothiuronium-dihydrobromid werden in 30 ml Pyridin unter Stickstoffatmosphäre 16 h gekocht. Der nach Eindampfen der Losung i. Vak. erhaltene Rückstand wird mit Ethylacetat/Methanol/konz. Ammoniak gesättigt mit Ammoniumchlorid (50:48:2) als

Eluent an Kieselgel chromatographisch gereinigt.

Die Hauptfraktion wird i.Vak. eingedampft. in 10 ml ges. Kaliumcarbonat aufgenommen und mit 2 x 20 ml Isopropanol extrahiert. Nach Trocknen und Eindampfen i.Vak. ergibt die organische Phase die Titelverbindung als farblosen, amorphen Feststoff.

Ausbeute: 0,19 g (42 %)
$C_{23}H_{29}N_9O$ (447,54)
$^1$H-NMR-Daten ($CD_3OD$, TMS als interner Standard)
$\delta$ = 1,8 - 2,2 (m) 4 H,
2,5 - 3,4 (m) 12 H,
5,0 (breit) 5 H, austauschbar mit $D_2O$,
6,9 (s) 1 H,
7,5 - 8,3 (m) 7 H ppm.

Beispiel 10

N$^1$-[3-[4-(-Methyl-6-oxo-1,4,5,6-tetrahydropyridazin-3-yl)phenoxy]propyl]-N$^2$-[3-(1H-imidazol-4-yl)propyl]-guanidin

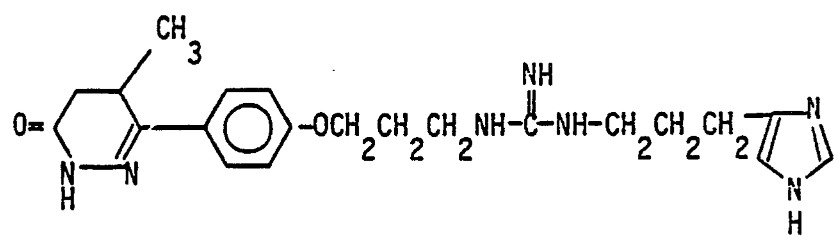

a) N$^1$-Benzoyl-N$^2$-[3-[4-(4-Methyl-6-oxo-1,4,5,6-tetrahydropyridazin-3-yl) phenoxy]propyl]-thioharnstoff

6,55 g (40 mmol) Benzoylisothiocyanat in 20 ml Dichlormethan werden während 20 Minuten tropfenweise zu einer Suspension von 10,45 g (40 mmol) 6-[4-(3-Aminopropoxy)phenyl]-4,5-dihydro-5-Methyl-3(2H)-pyridazinon in 100 ml Dichlormethan gegeben.
Nach 30 minütigem Nachrühren wird die Lösung im Vakuum eingedampft und der Rückstand durch Verrühren mit 30 ml Acetonitril kristallisiert.

Ausbeute: 11,38 g (67 %)
Schmp. 172 - 176 °C
$C_{22}H_{24}N_4O_3S$ (424,52)

b) N-[3-[4-(4-Methyl-6-oxo-1,4,5,6-tetrahydropyridazin-3-yl) phenoxy]propyl] -thioharnstoff

Erhalten analog zu Beispiel 6b) aus 8,0 g (18,8 mmol) N$^1$-Benzoyl-N$^2$-[3-[4 -(4-methyl-6-oxo-1,4,5,6-tetrahydropyridazin-3-yl) phenoxy]propyl]-thioharnstoff und 5,2 g (38 mmol) Kaliumcarbonat.

Ausbeute 5,3 g (88 %)
Farblose Kristalle vom Schmp. 196 - 198 °C
$C_{15}H_{20}N_4O_2S$ (320,41)

c) N-[3-[4-(4-Methyl-6-oxo-1,4,5,6-tetrahydropyridazin-3-yl) phenoxy]propyl] -S-methyl-isothiuroniumjodid

Hergestellt analog zu Beispiel 7c) aus 3,7 g (11,5 mmol) N-[3-[4-(4 Methyl-6-oxo-1,4,5,6-tetrahydropyridazin-3-yl) phenoxy]propyl]-thioharnstoff und 0,80 ml (12 mmol) Methyljodid. Man erhält 3,4 g (67 %) eines gelblichen, amorphen Feststoffs.

$C_{16}H_{23}JN_4O_2S$ (462,34)

d) N[1]-[3-[4-(4-Methyl-6-oxo-1,4,5,6-tetrahydropyridazin-3-yl) phenoxy]propyl] -N[2]-[3-(1H-imidazol-4-yl)-propyl]-guanidin

Aus 1,00 g (2,2 mmol) N-[3-[4-(4-Methyl-6-oxo-1,4,5,6-tetrahydropyridazin-3-yl) phenoxy]propyl]-S-methyl-isothiuroniumjodid und 0,30 g (2,4 mmol) 3-(1H-Imidazol4-yl)propylamin erhält man analog zu Beispiel 7d) 0,24 g (27 %) eines gelblichen amorphen Feststoffs.

$C_{21}H_{29}N_7O_2$ (411,51)

$^1$H-NMR-Daten ($CD_3OD$, TMS als interner Standard)
$\delta$ = 1,0 - 1,3 (2d) 3H
1,7 - 2,2 (m) 4H
2,5 - 2,8 (m) 4H
3,0 - 3,5 (m) 5H
4,2 (t) 2H
5,0 (breit) 5H, austauschb.m. $D_2O$
6,9 (s) 1H
7,0 - 7,2 (m) 2H
7,7 (s) 1H
7,8 - 8,0 (m) 2H ppm

Beispiel 11

N[1]-[3-[4-(4-Methyl-6-oxo-1,4,5,6-tetrahydropyridazin-3-yl)phenyl]propyl] N[2]-[3-(1H imidazol-4-yl)propyl]-guanidin

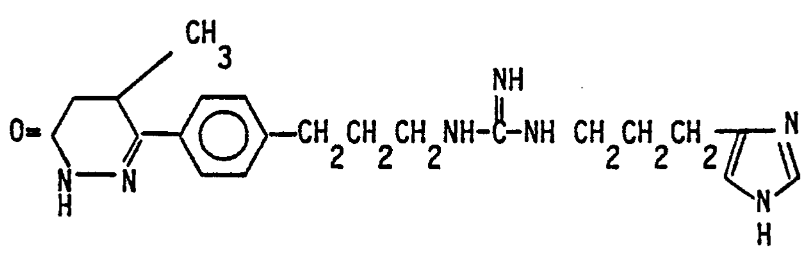

Analog zu Beispiel 7d) erhält man aus 1,00 g (2,2 mmol) N-[3-[4-(4-Methyl-6-oxo -1,4,5,6-tetrahydropyridazin-3-yl)phenyl]propyl]-S-methyl-isothiuroniumjodid und 0,28 g (2,2 mmol) 3-(1H-Imidazol-4-yl)propylamin 0,44 g (51 %) eines amorphen, erstarrten Schaums.

$C_{21}H_{29}N_7O$ (395,50)

$^1$H-NMR-Daten($CD_3OD$, TMS als interner Standard)
$\delta$ = 1,0 - 1,3 (2d) 3H

1,7 - 2,1 (m) 4H
2,5 - 2,9 (m) 6H
3,0 - 3,4 (m) 5H
5,0 (breit) 5H, austauschb.m. $D_2O$
6,9 (s) 1H
7,3 - 8,0 (m) 5H ppm

## Ansprüche

1. Dihydropyridazinon-Derivate der allgemeinen Formel I

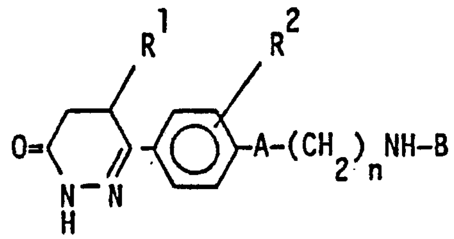

(I)

in der $R^1$ ein Wasserstoffatom oder eine gerad- oder verzweigtkettige $C_1$-$C_4$-Alkylgruppe bedeutet, $R^2$ für ein Wasserstoffatom, eine gerad- oder verzweigtkettige $C_1$-$C_4$-Alkylgruppe, eine $C_1$-$C_4$-Alkoxygruppe, ein Halogenatom, eine Nitrogruppe oder eine Aminogruppe steht, A für ein Sauerstoffatom, die Gruppe -NH- oder $-\underset{\underset{O}{\|}}{C}-NH-$, einen in den Positionen 1 und 4 verknüpften Imidazolring oder eine Einfachbindung steht, B ein Wasserstoffatom oder die Gruppierung

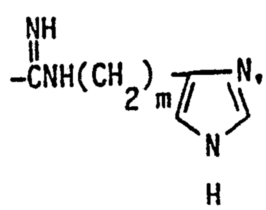

darstellt, in der m den Wert 2 oder 3 hat, und n eine ganze Zahl von 0 bis 6 ist, sowie die physiologisch annehmbaren Salze davon.

2. Dihydropyridazinon-Derivate nach Anspruch 1, dadurch **gekennzeichnet,** daß $R^1$ für ein Wasserstoffatom oder eine gerad- oder verzweigtkettige $C_1$-$C_4$-Alkylgruppe steht, $R^2$ ein Wasserstoffatom, eine gerad- oder verzweigtkettige $C_1$-$C_4$-Alkylgruppe, eine $C_1$-$C_4$-Alkoxygruppe, ein Halogenatom, eine Nitrogruppe oder eine Aminogruppe bedeutet, A für ein Sauerstoffatom, die Gruppe -NH- oder $-\underset{\underset{O}{\|}}{C}-NH;$ einen in den Positionen 1 und 4 verknüpften Imidazolring oder eine Einfachbindung steht, B ein Wasserstoffatom ist und n eine ganze Zahl von 0 bis 6 ist.

3. Dihydropyridazinon-Derivate nach Anspruch 1, dadurch **gekennzeichnet,** daß $R^1$ ein Wasserstoffatom oder eine gerad-oder verzweigtkettige $C_1$-$C_4$-Alkylgruppe bedeutet, $R^2$ für ein Wasserstoffatom, eine gerad- oder verzweigtkettige $C_1$-$C_4$-Alkylgruppe, eine $C_1$-$C_4$-Alkoxygruppe, ein Halogenatom, eine Nitrogruppe oder eine Aminogruppe steht, A für ein Sauerstoffatom, die Gruppe -NH- oder $-\underset{\underset{O}{\|}}{C}-NH-$, einen in den Positionen 1 und 4 verknüpften Imidazolring oder eine Einfachbindung steht, B die Gruppierung

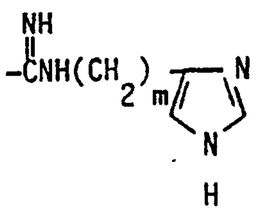

ist, in der m den Wert 2 oder 3 hat, und n eine ganze Zahl von 0 bis 6 ist.

4. Dihydropyridazinon-Derivate nach Anspruch 1, dadurch **gekennzeichnet**, daß $R^1$ für eine gerad- oder verzweigtkettige $C_1$-$C_4$-Alkylgruppe, insbesondere für eine Methylgruppe, steht, $R^2$ ein Wasserstoffatom bedeutet, A für ein Sauerstoffatom, die Gruppe -NH-oder - $\underset{\underset{O}{\|}}{C}$ -NH-, einen in den Positionen 1 und 4 gebundenen Imidazolring oder eine Einfachbindung steht, B ein Wasserstoffatom ist und n eine ganze Zahl zwischen 0 und 6 ist.

5. Dihydropyridazinon-Derivate nach Anspruch 1, dadurch **gekennzeichnet**, daß $R^1$ für ein Wasserstoffatom oder eine gerad- oder verzweigtkettige $C_1$-$C_4$-Alkylgruppe, insbesondere eine Methylgruppe, steht und $R^2$ ein Wasserstoffatom bedeutet, A ein Sauerstoffatom, die Gruppe -NH- oder - $\underset{\underset{O}{\|}}{C}$ -NH-, einen in den Positionen 1 und 4 verknüpften Imidazolring oder eine Einfachbindung darstellt, B die Gruppierung

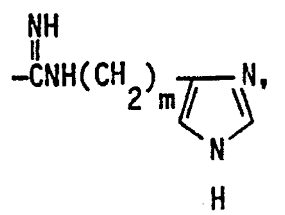

ist, in der m den Wert 2 oder 3, insbesondere 3, hat, und n eine ganze Zahl von 0 bis 6 ist.

6. 6-[4-[4-(3-Aminopropyl)-1H-imidazol-1-yl]phenyl)-4,5-dihydro-5-methyl-3(2H)-pyridazinon.

7. $N^1$-[3-[N-[4-(4-Methyl-6-oxo-1,4,5,6-tetrahydropyridazin-3-yl)phenyl] carboxamido]propyl]-$N^2$-[3-(1H-imidazol-4-yl)propyl]-guanidin.

8. $N^1$-[4-(4-Methyl-6-oxo-1,4,5,6-tetrahydropyridazin-3-yl)phenyl]-$N^2$-[3-(1H-imidazol-4-yl)propyl]-guanidin.

9. Verfahren zur Herstellung von Dihydropyridazinon-Derivaten der allgemeinen Formel I

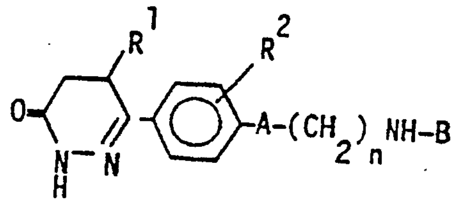

(I)

in der $R^1$ ein Wasserstoffatom oder eine gerad- oder verzweigtkettige $C_1$-$C_4$-Alkylgruppe bedeutet, $R^2$ für ein Wasserstoffatom, eine gerad- oder verzweigtkettige $C_1$- $C_4$ Alkylgruppe, eine $C_1$-$C_4$ Alkoxygruppe, ein Halogenatom, eine Nitrogruppe oder eine Aminogruppe steht, A für ein Sauerstoffatom, die Gruppe -NH- oder - $\underset{\underset{O}{\|}}{C}$ -NH-, einen in den Positionen 1 und 4 verknüpften Imidazolring oder eine Einfachbindung steht, B ein Wasserstoffatom oder die Gruppierung

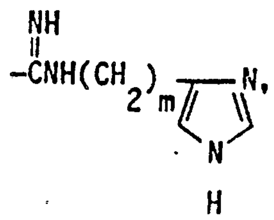

darstellt, in der m den Wert 2 oder 3 hat, und n eine ganze Zahl von 0 bis 6 ist, sowie der physiologisch annehmbaren Salze davon, dadurch **gekennzeichnet,** daß man

a) zur Herstellung von Verbindungen der allgemeinen Formel I, bei denen $R^1$, $R^2$, A und n wie oben definiert sind und B für ein Wasserstoffatom steht,

$a_1$) von einer Phthalimidverbindung der allgemeinen Formel II

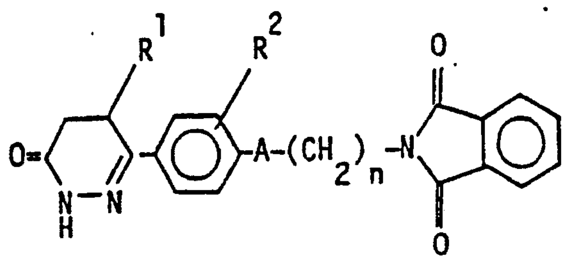

(II)

in der $R^1$, $R^2$, A und n die oben angegebenen Bedeutungen besitzen, durch Umsetzung mit Hydrazin oder einem chemischen Äquivalent davon, einem aliphatischen primären Amin oder einer Säure die Phthalimidgruppe abspaltet und dadurch in eine Verbindung der allgemeinen Formel I überführt oder

$a_2$) eine Verbindung der allgemeinen Formel III

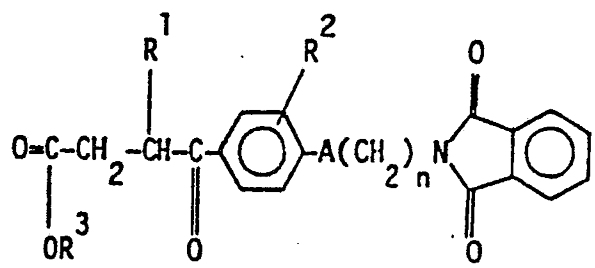

(III)

in der $R^1$, $R^2$, A und n die oben angegebenen Bedeutungen haben und $R^3$ für eine gegebenenfalls substituierte $C_1$-$C_4$-Alkylgruppe oder ein Wasserstoffatom steht, mit Hydrazin oder einem chemischen Äquivalent davon unter gleichzeitigem Ringschluß zum Py ridazinonring und Hydrazinolyse der Phthalimidschutzgruppe und unter Erhalt einer Verbindung der allgemeinen Formel I umsetzt oder

b) zur Herstellung von Verbindungen der allgemeinen Formel I, bei denen $R^1$, $R^2$, A und n wie oben definiert sind und B für die Gruppierung

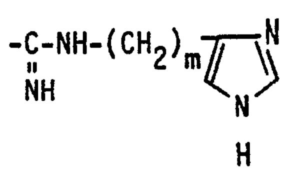

steht, in der m den Wert 2 oder 3 hat,

$b_1$) eine Verbindung der allgemeinen Formel IV

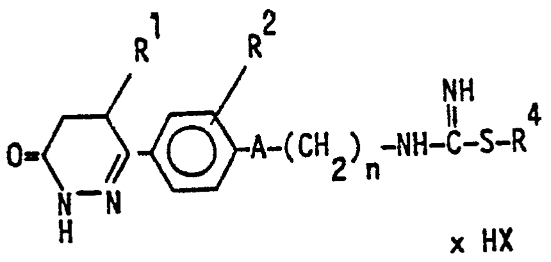

(IV)

in der $R^1$, $R^2$, A und n wie oben definiert sind, $R^4$ für eine gegebenenfalls substituierte $C_1$-$C_4$-Alkylgruppe steht und X ein Halogenatom oder die Gruppe $-OSO_2OR^4$ bedeutet, mit einem m-(Imidazol-4-yl)alkylamin der allgemeinen Formel V

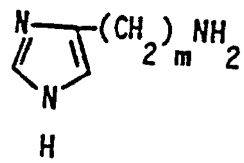

(V)

in der m den Wert 2 oder 3 hat, zu einer Verbindung der allgemeinen Formel I umsetzt oder

$b_2$) eine Verbindung der allgemeinen Formel VI

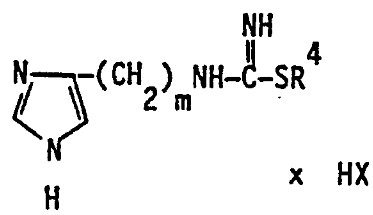

(VI)

in der m, $R^4$ und X wie oben definiert sind, mit einer Verbindung der allgemeinen Formel Ia

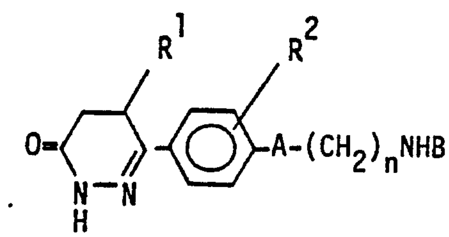

(Ia)

in der $R^1$, $R^2$, A und n die oben angegebenen Bedeutungen haben und B für ein Wasserstoffatom steht, zu einer Verbindung der allgemeinen Formel I umsetzt

und daß man gegebenenfalls die nach $a_1$) bis $a_2$) oder $b_1$) bis $b_2$) erhaltene Verbindung in an sich bekannter Weise in ihr physiologisch annehmbares Salz umwandelt.

10. Arzneimittel, dadurch **gekennzeichnet**, daß es eine Verbindung nach einem der Ansprüche 1 bis 8 zusammen mit mindestens einem inerten, pharmazeutisch annehmbaren Träger oder Verdünnungsmittel enthält.

Patentansprüche für folgende Vertragsstaaten : ES, GR

1. Verfahren zur Herstellung von Dihydropyridazinon-Derivaten der allgemeinen Formel 1

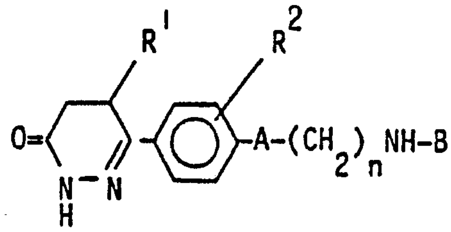

(I)

in der $R^1$ ein Wasserstoffatom oder eine gerad- oder verzweigtkettige $C_1$-$C_4$-Alkylgruppe bedeutet, $R^2$ für ein Wasserstoffatom, eine gerad- oder verzweigtkettige $C_1$-$C_4$-Alkylgruppe, eine $C_1$-$C_4$-Alkoxygruppe, ein Halogenatom, eine Nitrogruppe oder eine Aminogruppe steht, A für ein Sauerstoffatom, die Gruppe -NH- oder $-\underset{\underset{O}{\|}}{C}-NH-$,

einen in den Positionen 1 und 4 verknüpften Imidazolring oder eine Einfachbindung steht, B ein Wasserstoffatom oder die Gruppierung

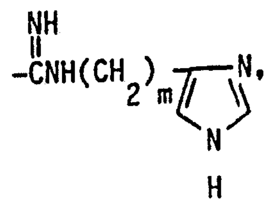

darstellt, in der m den Wert 2 oder 3 hat, und n eine ganze Zahl von 0 bis 6 ist, sowie der physiologisch annehmbaren Salze davon, dadurch **gekennzeichnet,** daß man

a) zur Herstellung von Verbindungen der allgemeinen Formel I, bei denen $R^1$, $R^2$, A und n wie oben definiert sind und B für ein Wasserstoffatom steht,

$a_1$) von einer Phthalimidverbindung der allgemeinen Formel II

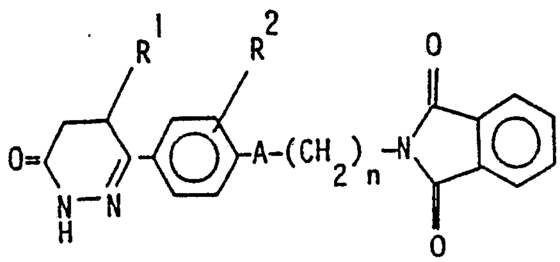

(II)

in der $R^1$, $R^2$, A und n die oben angegebenen Bedeutungen besitzen, durch Umsetzung mit Hydrazin oder einem chemischen Äquivalent davon, einem aliphatischen primären Amin oder einer Säure die Phthalimidgruppe abspaltet und dadurch in eine Verbindung der allgemeinen Formel I überführt oder

$a_2$) eine Verbindung der allgemeinen Formel III

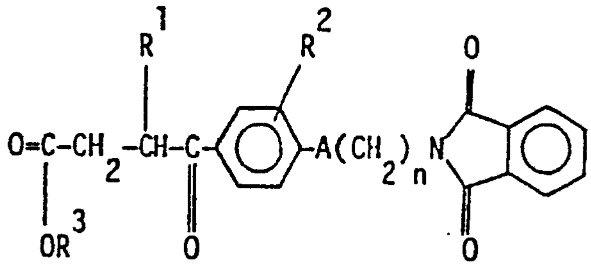

(III)

$R^1$, $R^2$, A und n die oben angegebenen Bedeutungen haben und $R^3$ für eine gegebenenfalls substituierte $C_1$-$C_4$-Alkylgruppe oder ein Wasserstoffatom steht, mit Hydrazin oder einem chemischen Äquivalent davon unter gleichzeitigem Ringschluß zum Py ridazinonring und Hydrazinolyse der Phthalimidschutzgruppe und unter Erhalt einer Verbindung der allgemeinen Formel I umsetzt oder

b) zur Herstellung von Verbindungen der allgemeinen Formel I, bei denen $R^1$, $R^2$, A und n wie oben definiert sind und B für die Gruppierung

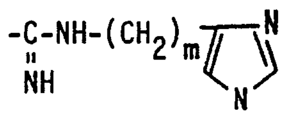

steht, in der m den Wert 2 oder 3 hat,

$b_1$) eine Verbindung der allgemeinen Formel IV

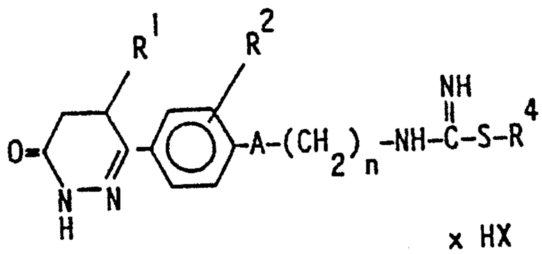

(IV)

in der $R^1$, $R^2$, A und n wie oben definiert sind, $R^4$ für eine gegebenenfalls substituierte $C_1$-$C_4$-Alkylgruppe steht und X ein Halogenatom oder die Gruppe $-OSO_2OR^4$ bedeutet, mit einem m-(Imidazol-4-yl)alkylamin der allgemeinen Formel V

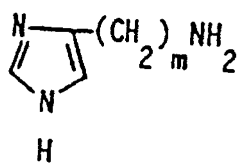

(V)

in der m den Wert 2 oder 3 hat, zu einer Verbindung der allgemeinen Formel I umsetzt oder

$b_2$) eine Verbindung der allgemeinen Formel VI

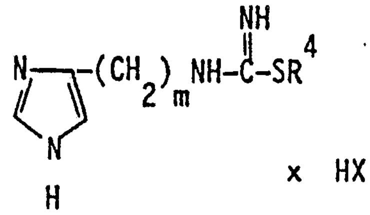

(VI)

in der m, $R^4$ und X wie oben definiert sind, mit einer Verbindung der allgemeinen Formel Ia

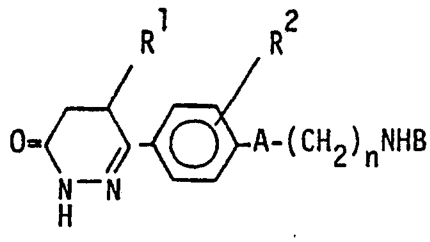

(Ia)

in der $R^1$, $R^2$, A und n die oben angegebenen Bedeutungen haben und B für ein Wasserstoffatom steht, zu einer Verbindung der allgemeinen Formel I umsetzt

und daß man gegebenenfalls die nach $a_1$) bis $a_2$) oder $b_1$) bis $b_2$) erhaltene Verbindung in an sich bekannter Weise in ihr physiologisch annehmbares Salz umwandelt.

2. Verfahren nach Anspruch 1 zur Herstellung von Verbindungen, bei denen $R^1$ für ein Wasserstoffatom oder eine gerad- oder verzweigtkettige $C_1$-$C_4$-Alkylgruppe steht, $R^2$ ein Wasserstoffatom, eine gerad- oder verzweigtkettige $C_1$-$C_4$-Alkylgruppe, eine $C_1$-$C_4$-Alkoxygruppe, ein Halogenatom, eine Nitrogruppe oder eine Aminogruppe bedeutet, A für ein Sauerstoffatom, die Gruppe -NH- oder $-\underset{\underset{O}{\|}}{C}-NH-$, einen in den Positionen 1 und 4 verknüpften Imidazolring oder eine Einfachbindung steht, B ein Wasserstoffatom ist und n eine ganze Zahl von 0 bis 6 ist.

3. Verfahren nach Anspruch 1 zur Herstellung von Verbindungen, bei denen $R^1$ ein Wasserstoffatom oder eine gerad- oder verzweigtkettige $C_1$-$C_4$-Alkylgruppe bedeutet, $R^2$ für ein Wasserstoffatom, eine gerad- oder verzweigtkettige $C_1$-$C_4$-Alkylgruppe, eine $C_1$-$C_4$-Alkoxygruppe, ein Halogenatom, eine Nitrogruppe oder eine Aminogruppe steht, A für ein Sauerstoffatom, die Gruppe -NH- oder $-\underset{\underset{O}{\|}}{C}-NH-$, einen in den Positionen 1 und 4 verknüpften Imidazolring oder eine Einfachbindung steht, B die Gruppierung

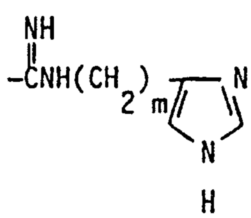

ist, in der m den Wert 2 oder 3 hat, und n eine ganze Zahl von 0 bis 6 ist.

4. Verfahren nach Anspruch 1 zur Herstellung von Verbindungen, bei denen $R^1$ für eine gerad- oder verzweigtkettige $C_1$-$C_4$-Alkylgruppe, insbesondere für eine Methylgruppe, steht, $R^2$ ein Wasserstoffatom bedeutet, A für ein Sauerstoffatom, die Gruppe -NH- oder $-\underset{\underset{O}{\|}}{C}-NH-$, einen in den Positionen 1 und 4 gebundenen Imidazolring oder eine Einfachbindung steht, B ein Wasserstoffatom ist und n eine ganze Zahl zwischen 0 und 6 ist.

5. Verfahren nach Anspruch 1 zur Herstellung von Verbindungen, bei denen $R^1$ für ein Wasserstoffatom oder eine gerad- oder verzweigtkettige $C_1$-$C_4$-Alkylgruppe, insbesondere eine Methylgruppe, steht und $R^2$ ein Wasserstoffatom bedeutet, A ein Sauerstoffatom, die Gruppe -NH- oder $-\underset{\underset{O}{\|}}{C}-NH-$,

einen in den Positionen 1 und 4 verknüpften Imidazolring oder eine Einfachbindung darstellt, B die Gruppierung

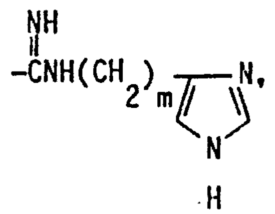

ist, in der m den Wert 2 oder 3, insbesondere 3, hat, und n eine ganze Zahl von 0 bis 6 ist.

6. Verfahren nach Anspruch 1 zur Herstellung von 6-4-4-(3-Aminopropyl)-1H-imidazol-1-yl]phenyl]-4,5-dihydro-5-methyl-3(2H)-pyridazinon.

7. Verfahren nach Anspruch 1 zur Herstellung von $N^1$-[3-[N-[4-(4-Methyl-6-oxo-1,4,5,6-tetrahydropyridazin-3-yl)phenyl] carboxamido]propyl]$N^2$-[3-(1H-imidazol-4-yl)propyl] -guanidin.

8. Verfahren nach Anspruch 1 zur Herstellung von $N^1$-[4-(4-Methyl-6-oxo-1,4,5,6-tetrahydropyridazin-3-yl)phenyl]-$N^2$-[3-(1H-imidazol-4-yl)propyl]-guanidin.

Europäisches Patentamt

# EUROPÄISCHER RECHERCHENBERICHT

Nummer der Anmeldung

EP 88101620.8

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | DE - A1 - 2 157 453 (BASF)<br>* Anspruch 1; Tabelle 1; Beispiel 1 *<br>-- | 1 | C 07 D 403/12<br>C 07 D 403/14<br>C 07 D 237/04<br>A 61 K 31/50 |
| X | CHEMICAL ABSTRACTS, Band 101, Nr. 11, 10. September 1984, Columbus, Ohio, USA<br>MITSUI TOATSU CHEMICALS, INC. "Pyridazinone derivatives." Seite 645, Spalte 1, Zusammenfassung-Nr. 90 957y<br>& Jpn. Kokai Tokkyo Koho JP 59 53,479 [84 53,479]<br>-- | 1,10 | |
| X | CHEMICAL ABSTRACTS, Band 97, Nr. 7, 16. August 1982, Columbus, Ohio, USA<br>MITSUI TOATSU CHEMICALS, INC. "Pyridazinone derivatives." Seite 653, Spalte 2, Zusammenfassung-Nr. 55 827h<br>& Jpn. Kokai Tokkyo Koho JP 82 46,966<br>-- | 1 | RECHERCHIERTE SACHGEBIETE (Int. Cl.4)<br>C 07 D 403/00<br>C 07 D 237/00 |

Der vorliegende Recherchenbericht wurde für alle Patentansprüche erstellt.

| Recherchenort | Abschlußdatum der Recherche | Prüfer |
|---|---|---|
| WIEN | 03-10-1988 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

**EUROPÄISCHER RECHERCHENBERICHT**

Nummer der Anmeldung



| EINSCHLÄGIGE DOKUMENTE | | | EP 88101620.8 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | CHEMICAL ABSTRACTS, Band 107, Nr. 3, 20. Juli 1987, Columbus, Ohio, USA<br>MORISAWA, YASUHIRO et al. "6-Phenylpyridazine derivatives as cardiovascular agents." Seite 615, Spalte 1, Zusammenfassung-Nr. 23 352h<br>& Jpn. Kokai Tokkyo Koho JP 62 53, 972 [87 53, 972]<br>-- | 1 | |
| X | CHEMICAL ABSTRACTS, Band 90, Nr. 21, 21. Mai 1979, Columbus, Ohio, USA<br>NAKAO, TORU et al. "6-(p-Substituted phenyl)-4,5-dihydro-3(2H)--pyridazinones." Seite 606, Spalte 2, Zusammenfassung-Nr. 168 635t<br>& Jpn. Kokai Tokkyo Koho 78,124,279<br>-- | 1 | RECHERCHIERTE SACHGEBIETE (Int. Cl.4) |
| X | CHEMICAL ABSTRACTS, Band 100, Nr. 7, 13. Februar 1984, Columbus, Ohio, USA<br>TEIKOKU HORMONE MFG. CO., LTD. "Pyridazinone derivatives." Seite 582, Spalte 2, Zusammenfassung-Nr. 51 594n<br>& Jpn. Kokai Tokkyo Koho JP 58,146,570 [83,146,570]<br>-- | 1 | |

Der vorliegende Recherchenbericht wurde für alle Patentansprüche erstellt.

| Recherchenort | Abschlußdatum der Recherche | Prüfer |
|---|---|---|
| WIEN | 03-10-1988 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

-3-

EP 88101620.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2 - 0 178 189 (SANKYO)<br>* Zusammenfassung; Ansprüche 1,33 *<br>---- | 1,10 | |
| | | | RECHERCHIERTE SACHGEBIETE (Int. Cl.4) |
| | | | |

Der vorliegende Recherchenbericht wurde für alle Patentansprüche erstellt.

| Recherchenort | Abschlußdatum der Recherche | Prüfer |
|---|---|---|
| WIEN | 03-10-1988 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82